(19) Europäisches Patentamt
European Patent Office
Office européen des brevets

(11) EP 0 779 030 B1

(12) EUROPÄISCHE PATENTSCHRIFT

(45) Veröffentlichungstag und Bekanntmachung des Hinweises auf die Patenterteilung:
**18.07.2001 Patentblatt 2001/29**

(51) Int Cl.[7]: **A01N 43/836**
// (A01N43/836, 43:90, 43:80, 37:38)

(21) Anmeldenummer: **96810844.9**

(22) Anmeldetag: **03.12.1996**

(54) **Pflanzenschutzmittel**

Plant protecting agents

Compositions phytosanitaires

(84) Benannte Vertragsstaaten:
**AT BE CH DE DK ES FR GB GR IE IT LI LU NL PT SE**

(30) Priorität: **11.12.1995 CH 349595**

(43) Veröffentlichungstag der Anmeldung:
**18.06.1997 Patentblatt 1997/25**

(73) Patentinhaber: **Syngenta Participations AG**
**4058 Basel (CH)**

(72) Erfinder: **Ruess, Wilhelm**
**4148 Pfeffingen (CH)**

(56) Entgegenhaltungen:
**EP-A- 0 313 512**

EP 0 779 030 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft neue pflanzenschützende Wirkstoff-Gemische mit synergistisch gesteigerter Wirkung, enthaltend mindestens zwei Wirkstoff-Komponenten zusammen mit einem geeigneten Trägermaterial, worin die Komponente I eine Verbindung mit pflanzenimmunisierender Wirkung der Formel I ist

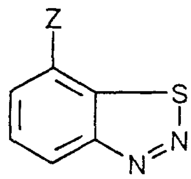

I

worin
Z COOH oder ein Salz davon, CO-$OC_1$-$C_4$-Alkyl oder CO-$SC_1$-$C_4$-Alkyl bedeutet; und worin die Komponente II ein Handelsprodukt ist ausgewählt aus der Gruppe

A) 4-[3-(4-Chlorphenyl)-3-(3,4-dimethyoxphenyl)acryloyl]morpholine, ("Dimethomorph"), (Referenz: C. Tomlin (Editor): The Pesticide Manual, 10th edition, Farnham, UK, 1994, pages 351-2);
B) 5-Methyl-1,2,4-triazolo[3,4,b][1,3]benzothiazol, ("Tricyclazol"), (Referenz: C. Tomlin (Editor): The Pesticide Manual, 10th edition, Farnham, UK, 1994, pages 1017-8).

Die Erfindung betrifft auch Salze und Metallkomplexe der Verbindungen I und II.
Unter den Verbindungen der Formel I sind diejenigen bevorzugt, worin
Z COOH (Verbindung IA) oder ein Salz davon, $COOCH_3$ (Verbindung IC) oder $COSCH_3$ (Verbindung ID) bedeutet.
Bevorzugte Salze sind Alkalimetall- und Erdalkalimetall-Salze, besonders Li, Na, K, Mg, oder Ca-Salze, ferner organische Salze, insbesondere solche von salzbildenden Aminen wie z.B. Trimethylamin, Triethylamin, N,N-Dimethylanilin, Pyridin, Triethanolamin, Morpholin.
Ganz besonders bevorzugt ist die Verbindung der Formel I, worin Z CO-$SCH_3$ bedeutet (Verbindung ID).
Es ist bekannt, dass Verbindungen der Formel I das in der Pflanze latent vorhandene eigene Abwehrsystem gegen pathogene mikrobielle Einflüsse aktivieren und somit die Pflanze vor pathogenen Erregem schützen können (EP-A-313,512).
Diese Verbindungen haben bei niedrigen Aufwandmengen keine direkte Wirkung auf die Schadorganismen, sondern sie bewirken eine Immunisierung der gesunden Pflanze gegen Krankheiten.
Der Nachteil bei der Bekämpfung von Pflanzenkrankheiten mit Verbindungen der Formel I besteht darin, dass die Wirkung bei niedrigen Aufwandmengen oft ungenügend ist.
Es wurde nun überraschenderweise gefunden, dass Verbindungen der Formel I im Gemisch mit einem der konventionellen Mikrobizide IIA bis IIB eine synergistisch gesteigerte Wirkung haben. Mit solchen Mischungen können Pflanzenkrankheiten bekämpft werden, indem einerseits die Pflanze durch die Aktivierung ihres eigenen Abwehrsystems gestärkt wird, und indem andererseits die Krankheitserreger zusätzlich direkt bekämpft werden. Gegenüber den herkömmlichen Methoden der Bekämpfung von Pflanzenkrankheiten sind unerwartet geringe Mengen an Wirkstoffen erforderlich.
Ein besonderer Vorteil der erfindungsgemässen Mischungen besteht ferner darin, dass, bedingt durch die völlig unterschiedlichen Wirkungsweisen der Komponenten I und II, drohenden Resistenzbildungen bei der Bekämpfung von Pflanzenkrankheiten wirksam vorgebeugt wird.
Die synergistisch gesteigerte Wirkung von Mischungen der Komponenten I und II zeigt sich beispielsweise in niedrigeren Aufwandmengen, längerer Wirkungsdauer und insgesamt grösseren Ernteerträgen. Aus der Summe der Wirkungen der Einzelkomponenten konnten derartige Steigerungen nicht erwartet werden.
Gegenstand der vorliegenden Erfindung ist auch ein Verfahren zum Schutz von Pflanzen vor Pflanzenkrankheiten, insbesondere vor Pilzbefall, durch Behandlung der Pflanzen, Teilen von Pflanzen oder ihrer Umgebung mit einer Komponente I und einer Komponente II in beliebiger Reihenfolge oder gleichzeitig.
Günstige Mischungsverhältnisse der beiden Wirkstoffe sind

I:IIA=1:30 bis 1:1, bevorzugt I:II = 1:30 bis 1:3 und 1:10 bis 1:3.
I:IIB = 1:20 bis 1:1, bevorzugt I:II = 1:5 bis 1:1 und 1:2.5 bis 1:1.

Die erfindungsgemässen Wirkstoffmischungen I+II besitzen sehr vorteilhafte Eigenschaften zum Schutz von

Pflanzen gegen Krankheitsbefall.
Mit den vorliegenden Wirkstoffmischungen können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben. Sie können auch als Beizmittel zur Behandlung von Pflanzenvermehrungsgut, insbesondere von Saatgut (Früchte, Knollen, Kömer) und Pflanzenstecklingen (z.B. Reis) zum Schutz vor Pilzinfektionen sowie gegen im Erdboden auftretende phytopathogene Pilze eingesetzt werden. Die erfindungsgemässen Wirkstoff-Gemische zeichnen sich durch besonders gute Pflanzenverträglichkeit und durch Umweltfreundlichkeit aus.

Die Wirkstoff-Gemische sind gegen die den folgenden Klassen angehörenden phytopathogenen Pilze wirksam: Ascomyceten (z.B. Venturia, Podosphaera, Erysiphe, Monilinia, Mycosphaerella, Uncinula); Basidiomyceten (z. B. die Gattung Hemileia, Rhizoctonia, Puccinia); Fungi imperfecti (z.B. Botrytis, Helminthosporium, Rhynchosporium, Fusarium, Septoria, Cercospora, Alternaria, Pyricularia und insbesondere Pseudocercosporella herpotrichoides); Oomyceten (z.B. Phytophthora, Peronospora, Bremia, Pythium, Plasmopara).

Als Zielkulturen für die hierin offenbarten Indikationsgebiete gelten im Rahmen dieser Erfindung z.B. folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Aepfel, Birnen, Pflaumen, Pfirsiche, Mandeln, Kirschen, Erdbeeren, Himbeeren und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Grapefruit, Mandarinen); Gemüsesorten (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen). Diese Aufzählung stellt keine Limitierung dar.

Besonders vorteilhaft sind die erfindungsgemässen Wirkstoffgemische für Anwendungen in Gemüse, Reis und Tabak; ferner in Kartoffeln, Reben, Rasen und Hopfen.
Besonders geeignet sind die Gemische

a) I+IIA für die Behandlung von Gemüse und Tabak gegen Oomyceten, insbesondere gegen Phytophthora, Peronospora, Bremia;
b) I+IIB für die Behandlung von Reis gegen Fungi imperfecti, insbesondere Pyricularia.

Die Wirkstoff-Gemische der Formeln I und II werden üblicherweise in Form von Zusammensetzungen verwendet. Die Wirkstoffe der Formeln I und II können gleichzeitig, können aber auch nacheinander am selben Tage auf die zu behandelnde Fläche oder Pflanze gegeben werden, zusammen mit gegebenenfalls weiteren in der Formulierungstechnik üblichen Trägerstoffen, Tensiden oder anderen applikationsfördernden Zusätzen.
Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoff-Gemisches das mindestens je einen dieser Wirkstoffe I und II enthält, ist das Aufbringen auf die oberirdischen Pflanzenteile, vor allem das Blattwerk (Blattapplikation). Anzahl der Applikationen und Aufwandmenge richten sich nach den biologischen und klimatischen Lebensbedingungen für den Erreger. Die Wirkstoffe können aber auch über den Erdboden oder über das Wasser durch das Wurzelwerk in die Pflanze gelangen (systemische Wirkung), indem man den Standort der Pflanze mit einer flüssigen Zubereitung tränkt (z.B. im Reisanbau) oder die Substanzen in fester Form in den Boden einbringt z.B. in Form von Granulat (Bodenapplikation). Die Verbindungen der Formeln I und II können auch zur Saatgutbehandlung auf Samenkörner aufgebracht werden (Coating), indem man die Knollen bzw. Körner entweder nacheinander in einer flüssigen Zubereitung eines Wirkstoffs tränkt oder sie mit einer bereits kombinierten feuchten oder trockenen Zubereitung beschichtet. Darüberhinaus sind in besonderen Fällen weitere Applikationsarten bei Pflanzen möglich, z.B. die gezielte Behandlung der Knospen oder der Fruchtstände.

Die Verbindungen der Kombination werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, oder durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen des Wirkstoffgemischs liegen im allgemeinen bei 50g bis 2kg AS/ha, insbesondere bei 100g bis 1000g AS/ha, besonders bevorzugt bei 250g bis 700g AS/ha. Für die Saatgutbehanlung sind die Aufwandmengen 0.5g-1000g, vorzugsweise 5g-100g AS pro 100 kg Saatgut.

Die Formulierungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethylether oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehl verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art der zu formulierenden Wirkstoffe der Formeln I und II nichtionogene, kation- und/oder anionaktive Tenside mit guten Emulgier-, Dispergier- und Netzeigenschaften in Betracht. Unter Tensiden sind auch Tensidgemische zu verstehen.

Besonders vorteilhafte, applikationsfördernde Zuschlagstoffe sind femer natürliche oder synthetische Phospholipide aus der Reihe der Kephaline und Lecithine, wie z.B. Phosphatidylethanolamin, Phosphatidylserin, Phosphatidylglycerin, Lysolecithin.

Die agrochemischen Zubereitungen enthalten in der Regel 0,1 bis 99%, insbesondere 0,1 bis 95% Wirkstoffe der Formeln I und II, 99,9 bis 1%, insbesondere 99,9 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die nachfolgenden Beispiele dienen der Illustration der Erfindung, wobei "Wirkstoff" ein Gemisch aus Verbindung I und Verbindung II in einem bestimmten Mischungs-Verhältnis bedeutet.

Während als Handelsware eher konzentrierte Mittel bevorzugt werden, verwendet der Endverbraucher in der Regel verdünnte Mittel.

Formulierungsbeispiele

| Spritzpulver | a) | b) | c) |
|---|---|---|---|
| Wirkstoff [I:II = 1:3(a), 1:2(b), 1:1(c)] | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Iphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Emulsions-Konzentrat | |
|---|---|
| Wirkstoff (I:II = 1:6) | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Verdünnung hergestellt werden, die sich im Pflanzenschutz, einsetzen lassen.

| Stäubemittel | a) | b) | c) |
|---|---|---|---|
| Wirkstoff [I:II = 1:6(a), 1:2(b), 1:10(c)]: | 5 % | 6 % | 4 % |
| Talkum | 95 % | - | - |
| Kaolin | - | 94 % | |
| Gesteinsmehl | - | - | 96 % |

Man erhält anwendungsfertige Stäubemittel, indem der Wirkstoff mit dem Träger vermischt und auf einer geeigneten Mühle vermahlen wird. Solche Pulver lassen sich auch zur Trockenbeize für Saatgut verwenden.

| Extruder Granulat | |
|---|---|
| Wirkstoff (I:II = 2:1) | 15 % |
| Na-Ligninsulfonat | 2 % |
| Carboxymethylcellulose | 1 % |
| Kaolin | 82 % |

Der Wirkstoff wird mit den Zusatzstoffen vermischt, vermahlen und mit Wasser angefeuchtet. Dieses Gemisch wird extrudiert und anschliessend im Luftstrom getrocknet.

| Umhüllungs-Granulat | |
|---|---|
| Wirkstoff (I:II = 1:10) | 8 % |
| Polyethylenglykol (Molekulargewicht 200) | 3 % |
| Kaolin | 89 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| Suspensions-Konzentrat | |
|---|---|
| Wirkstoff (I:II = 1:8) | 40% |
| Propylenglykol | 10 % |
| Nonylphenolpolyethylenglykolether (15 Mol Et-oxid) | 6 % |
| Na-Ligninsulfonat | 10 % |
| Carboxymethylcellulose | 1 % |
| Silikonöl (in Form 75%ig. wässriger Emulsion) | 1 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Verdünnung hergestellt werden können. Mit solchen Verdünnungen kann man lebende Pflanzen sowie pflanzliches Vermehrungsgut durch Besprühen, Begiessen oder Eintauchen behandeln und vor Mikoorganismen-Befall schützen.

Biologische Beispiele

Ein synergistischer Effekt liegt dann vor, wenn die Wirkung der Wirkstoffkombination grösser ist als die Summe der Wirkungen der Einzelkomponenten.

Die zu erwartende Wirkung E für eine gegebene Wirkstoff-Kombination gehorcht der sogenannten COLBY-Formel und kann wie folgt berechnet werden, (COLBY, S.R. "Calculating synergistic and antagonistic responses of herbicide combination". Weeds, Vol.15, Seiten 20-22; 1967):

ppm = Milligramm Wirkstoff (=WS) pro Liter Spritzbrühe
X = % Wirkung durch Wirkstoff I bei Anwendung von p ppm Wirkstoff

Y = % Wirkung durch Wirkstoff II bei Anwendung von q ppm Wirkstoff.

Die erwartete (additive) Wirkung der Wirkstoffe I+II bei Anwendung von p+q ppm Wirkstoff ist nach nach Colby

$$E = X + Y - \frac{X \cdot Y}{100}$$

Wenn die tatsächlich beobachtete Wirkung (O) grösser ist als die erwartete (E), so ist die Kombination in ihrer Wirkung überadditiv, d.h. es liegt ein synergistischer Effekt vor.
O/E = Synergiefaktor (SF).
In den folgenden Beispielen wird der Befall der unbehandelten Pflanzen gleich 100% gesetzt, was einer Wirkung von 0% entspricht.

Beispiel B-1: Wirkung gegen Phytophthora infestans auf Tomaten

a) Kurative Wirkung

Tomatenpflanzen der Sorte "Roter Gnom" werden nach dreiwöchiger Anzucht mit einer Zoosporensuspension des Pilzes besprüht und in einer Kabine bei 18 bis 20° und gesättigter Luftfeuchtigkeit inkubiert. Unterbruch der Befeuchtung nach 24 Stunden. Nach dem Abtrocknen der Pflanzen werden diese mit einer Brühe besprüht, die die als Spritzpulver formulierte Wirksubstanz in einer Konzentration von 200 ppm enthält. Nach dem Antrocknen des Spritzbelages werden die Pflanzen wieder in der Feuchtkabine während 4 Tagen aufgestellt. Anzahl und Grösse der nach dieser Zeit aufgetretenen typischen Blattflecken sind der Bewertungsmassstab für die Wirksamkeit der geprüften Substanzen.

b) Präventiv-Systemische Wirkung

Die als Spritzpulver formulierte Wirksubstanz wird in einer Konzentration von 60 ppm (bezogen auf das Bodenvolumen) auf die Bodenoberfläche von drei Wochen alten eingetopften Tomatenpflanzen der Sorte "Roter Gnom" gegeben. Nach dreitägiger Wartezeit wird die Blattunterseite der Pflanzen mit einer Zoosporensuspension von Phytophthora infestans besprüht. Sie wird dann 5 Tage in einer Sprühkabine bei 18 bis 20°C und gesättigter Luftfeuchtigkeit gehalten. Nach dieser Zeit bilden sich typische Blattflecken, deren Anzahl und Grösse zur Bewertung der Wirksamkeit der geprüften Substanzen dienen. Insbesondere Mischungen der Komponenten IA oder ID mit IIA liefern gute Ergebnisse.

Beispiel B-2: Wirkung gegen Peronospora tabacina an Tabakpflanzen

Tabakpflanzen (6 Wochen alt) werden mit einer formulierten Lösung des Wirkstoffes besprüht. Vier Tage nach der Behandlung werden die Pflanzen mit einer Sporangiensuspension des Pilzes inokuliert, 4-5 Tage bei hoher Luftfeuchtigkeit aufbewahrt und dann bei normaler Tag/Nacht Wechselfolge weiterinkubiert.
Die Beurteilung der Symptome in den Tests erfolgt aufgrund der mit Pilz befallenen Blattoberfläche. Der Befall der unbehandelten Pflanzen entspricht 0% Wirkung.

Komponente I: Verbindung ID (Benzothiadiazol-7-carbonsäure-thiomethylester)
Komponente II: Verbindung IIA (Dimethomorph)

| Vers. Nr | mg WS pro liter (ppm) | | I:II | % Wirkung | | SF |
|---|---|---|---|---|---|---|
| | WS ID | WS IIA | | O (gefunden) | E (erwartet) | O/E |
| 1 | 0.03 | | | 14 | | |
| 2 | 0.1 | | | 34 | | |
| 3 | 0.3 | | | 88 | | |
| 4 | | 0.3 | | 52 | | |
| 5 | | 1 | | 52 | | |
| 6 | 0.03 | 1 | 1:33 | 74 | 59 | 1.3 |

(fortgesetzt)

| Vers. Nr | mg WS pro liter (ppm) | | I:II | % Wirkung | | SF |
|---|---|---|---|---|---|---|
| | WS ID | WS IIA | | O (gefunden) | E (erwartet) | O/E |
| 7 | 0.1 | 0.3 | 1:3 | 92 | 68 | 1.4 |
| 8 | 0.1 | 1 | 1:10 | 95 | 68 | 1.4 |

Beispiel B-3: Wirkung gegen Colletotrichum lagenarium auf Cucumis sativus L.

a) Gurkenpflanzen werden nach 10-14 tägiger Anzucht mit einer aus Spritzpulver des Wirkstoffes hergestellten Spritzbrühe besprüht. Nach 3-4 Tagen werden die Pflanzen mit einer Sporensuspension ($1.0 . 10^5$ Sporen/ml) des Pilzes infiziert und für 30 Stunden bei hoher Luftfeuchtigkeit und einer Temperatur von 23°C inkubiert. Die Inkubation wird dann bei normaler Luftfeuchtigkeit und bei 22°C bis 23°C weitergeführt.
Die Beurteilung der Schutzwirkung erfolgt aufgrund des Pilzbefalls 7-10 Tage nach der Infektion.
b) Gurkenpflanzen werden nach 10-14 tägiger wöchiger Anzucht mit einer aus Spritzpulver des Wirkstoffes hergestellten Spritzbrühe durch Bodenapplikation behandelt. Nach 3-4 Tagen werden die Pflanzen mit einer Sporensuspension ($1.5 . 10^5$ Sporen/ml) des Pilzes infiziert und für 30 Stunden bei hoher Luftfeuchtigkeit und einer Temperatur von 23°C inkubiert. Die Inkubation wird dann bei normaler Luftfeuchtigkeit und bei 22°C weitergeführt. Die Beurteilung der Schutzwirkung erfolgt aufgrund des Pilzbefalls 7-10 Tage nach der Infektion.

Insbesondere Mischungen der Komponenten IA oder ID mit IIA liefern gute Ergebnisse.

Beispiel B-4: Wirkung gegen Pyricularia oryzae auf Reispflanzen

Etwa 2-wöchige Reispflanzen werden mitsamt der Erde um die Wurzel in ein mit Spritzbrühe gefülltes Gefäss gestellt. Nach 36 Tagen wird der Pilzbefall beurteilt. Der Befall der unbehandelten Pflanzen entspricht 0% Wirkung.

Komponente I: Verbindung ID (Benzothiadiazol-7-carbonsäure-thiomethylester)
Komponente II: Verbindung IIB (Tricyclazol)

| Vers. Nr | mg WS pro liter (ppm) | | I:II | % Wirkung | | SF |
|---|---|---|---|---|---|---|
| | WS ID | WS IIB | | O (gefunden) | E (erwartet) | O/E |
| 1 | 0.5 | | | 65 | | |
| 2 | 0.25 | | | 39 | | |
| 3 | 0.1 | | | 18 | | |
| 4 | 0.05 | | | 5 | | |
| 5 | | 1 | | 74 | | |
| 6 | | 0.5 | | 71 | | |
| 7 | | 0.25 | | 48 | | |
| 8 | | 0.1 | | 32 | | |
| 9 | 0.25 | 0.25 | 1:1 | 75 | 68 | 1.1 |
| 10 | 0.1 | 0.25 | 1:2.5 | 69 | 57 | 1.2 |
| 11 | 0.1 | 0.1 | 1:1 | 61 | 44 | 1.4 |
| 12 | 0.05 | 1 | 1:20 | 80 | 75 | 1.1 |
| 13 | 0.05 | 0.25 | 1:5 | 58 | 50 | 1.2 |

## Patentansprüche

1. Pflanzenschützendes Mittel mit synergistischer Wirkung gegen Krankheitsbefall, enthaltend mindestens zwei Wirkstoff-Komponenten, zusammen mit einem geeigneten Trägermaterial, worin die Komponente I eine Verbindung

mit pflanzenimmunisierender Wirkung der Formel I ist

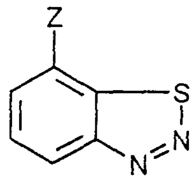

I

worin
Z COOH oder ein Salz davon, CO-OC$_1$-C$_4$-Alkyl oder CO-SC$_1$-C$_4$-Alkyl bedeutet; und worin die Komponente II eine Verbindung ist ausgewählt aus der Gruppe

A) 4-[3-(4-Chlorphenyl)-3-(3,4-dimethyoxphenyl)acryloyl]morpholine, ("Dimethomorph");
B) 5-Methyl-1,2,4-triazolo[3,4,b][1,3]benzothiazol, ("Tricyclazol").

**2.** Mittel gemäss Anspruch 1, worin das Gewichtsverhältnis I:IIA = 1:30 bis 1:1 beträgt.

**3.** Mittel gemäss Anspruch 1, worin das Gewichtsverhältnis I:IIB = 1:20 bis 1:1 beträgt.

**4.** Mittel gemäss Anspruch 1, worin in der Verbindung der Formel I
Z COOH (Verbindung IA) oder ein Salz davon, $COOCH_3$ (Verbindung IC) oder $COSCH_3$ (Verbindung ID) bedeutet.

**5.** Mittel gemäss Anspruch 4, worin in der Verbindung der Formel I Z CO-$SCH_3$ (Verbindung ID) bedeutet.

**6.** Mittel gemäss Anspruch 1, worin die Komponente II die Verbindung IIA ("Dimethomorph") ist.

**7.** Mittel gemäss Anspruch 1, worin die Komponente II die Verbindung IIB ("Tricyclazol") ist.

**8.** Verfahren zum Schutz von Pflanzen vor Pflanzenkrankheiten durch Behandlung der Pflanzen, Teilen von Pflanzen oder ihrer Umgebung mit einer Komponente I und einer Komponente II gemäss Anspruch 1, in beliebiger Reihenfolge oder gleichzeitig.

**9.** Verfahren gemäss Anspruch 8, worin in der Verbindung der Formel I
Z COOH (Verbindung IA) oder ein Salz davon, $COOCH_3$ (Verbindung IC) oder $COSCH_3$ (Verbindung ID) bedeutet.

**10.** Verfahren gemäss Anspruch 9, worin in der Verbindung der Formel I Z CO-$SCH_3$ (Verbindung ID) bedeutet.

**11.** Verfahren gemäss Anspruch 8 zur Behandlung von Gemüse und Tabak, worin die Komponente II die Verbindung IIA ist.

**12.** Verfahren gemäss Anspruch 8 zur Behandlung von Reis, worin die Komponente II die Verbindung IIB ist.

## Claims

**1.** A plant-protecting composition having synergistic action against disease infestation, comprising at least two active ingredient components together with a suitable carrier, wherein component I is a compound having plant-immunising action of formula I

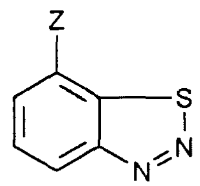

I

wherein
Z is COOH or a salt thereof, CO-OC$_1$-C$_4$alkyl or CO-SC$_1$-C$_4$alkyl;
and wherein component II is a compound selected from the group

A) 4-[3-(4-chlorophenyl)-3-(3,4-dimethoxyphenyl)acryloyl]morpholine ("dimethomorph");
B) 5-methyl-1,2,4-triazolo[3,4-b][1,3]benzothiazole ("tricyclazole").

**2.** A composition according to claim 1, wherein the ratio by weight of I:IIA is from 1:30 to 1:1.

**3.** A composition according to claim 1, wherein the ratio by weight of I:IIB is from 1:20 to 1:1.

**4.** A composition according to claim 1, wherein in the compound of formula I
Z is COOH (compound IA) or a salt thereof, $COOCH_3$ (compound IC) or $COSCH_3$ (compound ID).

**5.** A composition according to claim 4, wherein in the compound of formula I Z is CO-$SCH_3$ (compound ID).

**6.** A composition according to claim 1, wherein component II is compound IIA ("dimethomorph").

**7.** A composition according to claim 1, wherein component II is compound IIB ("tricyclazole").

**8.** A method of protecting plants against plant diseases by treating the plants, parts of the plants or their surroundings with a component I and a component II according to claim 1, in any desired sequence or simultaneously.

**9.** A method according to claim 8, wherein in the compound of formula I
Z is COOH (compound IA) or a salt thereof, $COOCH_3$ (compound IC) or $COSCH_3$ (compound ID).

**10.** A method according to claim 9, wherein in the compound of formula I Z is CO-$SCH_3$ (compound ID).

**11.** A method according to claim 8 for the treatment of vegetables and tobacco, wherein component II is compound IIA.

**12.** A method according to claim 8 for the treatment of rice, wherein component II is compound IIB.

**Revendications**

**1.** Produit phytosanitaire ayant une action synergique contre une infestation par une maladie, contenant au moins deux composants actifs, en même temps qu'un support approprié, où le composant I est un composé ayant une action à effet d'immunisation des plantes, de formule I

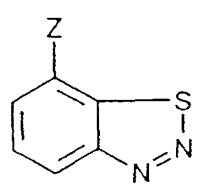

I

dans laquelle
Z est COOH ou un sel de ce dernier, un groupe CO-O(alkyle en C$_1$-C$_4$) ou CO-S(alkyle en C$_1$-C$_4$) ;

et où le composant II est un produit du commerce choisi dans l'ensemble comprenant :

A) la 4-[3-(4-chlorophényl)-3-(3,4-diméthoxyphényl)acryloyl]morpholine ("diméthomorph") ;
B) le 5-méthyl-1,2,4-triazolo[3,4,b][1,3]benzothiazole, ("tricyclazol").

**2.** Produit selon la revendication 1, dans lequel le rapport en poids I:IIA est de 1:30 à 1:1.

**3.** Produit selon la revendication 1, dans lequel le rapport en poids I:IIB est de 1:20 à 1:1.

**4.** Produit selon la revendication 1, dans lequel, dans le composé de formule I, Z est COOH (composé IA) ou un sel de ce dernier, $COOCH_3$ (composé IC) ou $COSCH_3$ (composé ID).

**5.** Produit selon la revendication 4, dans lequel, dans le composé de formule I, Z est $CO\text{-}SCH_3$ (composé ID).

**6.** Produit selon la revendication 1, dans lequel le composant II est le composé IIA ("diméthomorph").

**7.** Produit selon la revendication 1, dans lequel le composant II est le composé IIB ("tricyclazol").

**8.** Procédé pour protéger des végétaux contre des maladies des plantes, par traitement des plantes, de parties de plantes ou de leur environnement par un composant I et un composant II selon la revendication 1, dans un ordre quelconque ou simultanément.

**9.** Procédé selon la revendication 8, dans lequel, dans le composé de formule I, Z est COOH (composé IA) ou un sel de ce dernier, $COOCH_3$ (composé IC) ou $COSCH_3$ (composé ID).

**10.** Procédé selon la revendication 9, dans lequel, dans le composé de formule I, Z est $CO\text{-}SCH_3$ (composé ID).

**11.** Procédé selon la revendication 8 pour le traitement de légumes et de tabac, dans lequel le composant II est le composé IIA.

**12.** Procédé selon la revendication 8 pour le traitement du riz, dans lequel le composant II est le composé IIB.